# EUROPÄISCHE PATENTSCHRIFT

(11) **EP 2 651 313 B1**
(45) Veröffentlichungstag und Bekanntmachung des Hinweises auf die Patenterteilung: **01.10.2014**
(21) Anmeldenummer: 11802271.4
(22) Anmeldetag: 28.11.2011
(51) Int. Cl.: A61B 17/08, A61B 19/00

(54) **INSTRUMENT ZUM DEHNEN DER HAUT**
INSTRUMENT FOR STRETCHING THE SKIN
INSTRUMENT POUR DILATER LA PEAU

(30) Priorität: 15.12.2010 DE 102010054637
(43) Veröffentlichungstag der Anmeldung: 23.10.2013
(73) Patentinhaber: Fleischmann, Wilhelm, 79104 Freiburg (DE)
(72) Erfinder: Fleischmann, Wilhelm, 79104 Freiburg (DE)
(74) Vertreter: Patentanwälte Westphal, Mussgnug & Partner
(86) Internationale Anmeldenummer: PCT/EP2011/005959
(87) Internationale Veröffentlichungsnummer: WO 2012/079702

(56) Entgegenhaltungen:
- EP-A1- 1 340 461
- WO-A1-2010/092455
- WO-A2-96/18345
- DE-A1- 4 335 432
- DE-A1-102007 011 570
- SU-A1- 1 412 751
- US-A- 4 526 173
- US-A- 5 893 879

## Beschreibung

Die Erfindung betrifft ein Instrument zum Dehnen der Haut für den Verschluss von Wunden gemäß dem Oberbegriff des Patentan-spruchs 1.

Zum Schließen großflächiger Hautdefekte ist neben der Hauttransplantation insbesondere auch die Hautdehntechnik, das sogenannte Skin-Stretching, bekannt. Bei dem Skin-Stretching wird insbesondere ein Hautexpander bzw. Hautdistraktor verwendet, der wenigstens zwei Backen aufweist, die mit zugeordneten Haken in den einander gegenüberliegenden Wundrändern verankert werden. Die Backen werden mechanisch gegeneinander verstellt und aufeinander zu bewegt, wodurch auf die Haut eine Zugkraft ausgeübt wird, die die Haut dehnt und die Wundränder gegeneinander zusammenzieht. Durch eine über einen definierten Zeitraum wirkende Zugkraft wird die Haut über ihre natürliche Elastizitätsgrenze hinaus gedehnt, wodurch eine zusätzlich Gewebsproliferation erzeugt wird, sodass ein für den Verschluss der Wunde geeigneter Flächengewinn der körpereigenen Haut erreicht werden kann.

Für das Skin-Stretching geeignete Hautexpander sind in unterschiedlichsten Ausführungen bekannt. Die Backen können bspw. auf Führungen mittels einer Spindel gegeneinander bewegt werden (z. B. EP 0 797406 B1). Ebenso ist es bekannt, die Backen mittels Schiebern auf einer Rastverzahnung zu bewegen, die an einem flexiblen Band oder an einer starren Stange ausgebildet ist (z. B. EP 2 120 767 B1). Wie aus diesem Stand der Technik bekannt ist, können die Haken, die zur Verankerung der Backen an den Wundrändern in die Haut eingestochen werden, an den Backen selbst angeformt sein oder als Hakenmodule in die Backen einsetzbar sein. Bei dem Skin-Stretching mit den bekannten Instrumenten werden die Wundränder unter Dehnung der an die Wunde angrenzenden Hautbereiche in einem oder bei großflächigen Wunden auch in mehreren Schritten zusammengezogen, bis die entgegengesetzten Wundränder aneinander anliegen und vernäht werden können. Der teilweise sperrige und aufwändige Hautexpander bleibt daher über einen längeren Zeitraum, der unter Umständen einige Tage dauern kann, an der Wunde. Bei großflächigen Wunden ist es dabei unter Umständen auch erforderlich, mehrere Hautexpander nebeneinander einzusetzen.

US 5,893,879 A offenbart ein Instrument entsprechend dem Oberbegriff des Patentanspruchs 1.

Der Erfindung liegt die Aufgabe zugrunde, ein Instrument zum Dehnen der Haut für den Verschluss von Wunden zur Verfügung zu stellen, welches eine verbesserte Wundbehandlung ermöglicht und wirtschaftlicher im Einsatz ist.

Diese Aufgabe wird erfindungsgemäß gelöst durch ein Instrument mit den Merkmalen des Patentanspruchs 1.

Vorteilhafte Ausführungen der Erfindung sind in den rückbezogenen Unteransprüchen angegeben.

Der wesentliche Gedanke der Erfindung besteht darin, die in die Haut der Wundränder einzustechenden Haken jeweils an einem Träger anzuordnen, der lösbar in dem jeweiligen Backen des Hautexpanders gelagert ist. Nach dem Einstechen der Haken in die Wundränder werden die Backen mit den in die Backen aufgenommenen Trägern mittels des Hautexpanders gegeneinander bewegt, um die Wundränder unter Dehnung der Haut zu schließen. Wenn der Dehnungsvorgang mittels des Hautexpanders abgeschlossen ist, werden die an den einander gegenüberliegenden Wundrändern mittels der Haken verankerten Träger durch ein aufsetzbares Fixerelement in ihrer gegenseitigen Position fixiert. Der Hautexpander kann dann von den Trägern abgenommen werden, sodass die Wundränder nun nur durch die mittels der Fixierelemente miteinander verbundenen Träger und deren Haken zusammengehalten werden.

Das Instrument weist wesentliche Vorteile auf. Nachdem die Wundränder zusammengezogen und die Wunde geschlossen ist, können die an den Wundrändern fixierten Träger mittels der Fixierelemente fixiert werden und der Hautexpander kann abgenommen werden. Die Wundränder können durch die fixierten und verbleibenden Träger über einen längeren Zeitraum zusammengehalten werden, sodass ein Vernähen der Wundränder nicht erforderlich ist. Der abgenommene Hautexpander kann für weitere Operationen verwendet werden.

Bei großen Hautdefekten kann der Hautexpander auch anschließend verwendet werden, um weitere Träger mit Haken seitlich anschließend an die ersten Träger in den Wundrändern einzusetzen, um die Wunde schrittweise zu schließen. Der aufwändige Hautexpander kann hierbei als mehrfach verwendbares sterilisierbares Instrument ausgebildet sein.

Die Fixierelemente können in unterschiedlichen Dimensionen zur Verfügung stehen, um die in den einander gegenüberliegenden Wundrändern fixierten Träger in unterschiedlichem gegenseitigem Abstand zu halten. Kann die Wunde in einem einzigen Dehnungsschritt geschlossen werden, so werden Fixierelemente verwendet, die die einander gegenüberliegenden Träger in einem solchen Abstand fixieren, dass die Wundränder aneinander anliegend gehalten werden. Ist ein Wundverschluss in einem einzigen Dehnungsschritt nicht möglich, so können Fixierelemente verwendet werden, welche die einander gegenüberliegenden Träger in einem dem jeweiligen Dehnungsschritt entsprechenden Abstand fixiert halten. Der Hautexpander kann abgenommen und anderweitig verwendet werden, bis er wieder für den nächsten Dehnungsschritt an den Trägern angesetzt wird. Da die an der Wunde verbleibenden Träger und die Fixierelemente nur wenig Platz benötigen, kann die noch nicht vollständig geschlossene Wunde zwischenzeitlich bis zum nächsten Einsatz des Hautexpanders optimal versorgt werden. Insbesondere kann die Wunde zwischenzeitlich z. B. durch eine Vakuumversiegelung abgedeckt werden, die über die an den Wundrändern verankerten Träger und die Fixierelemente gelegt wird.

In einer vorteilhaften Ausführung sind die Träger als quer zur Zugrichtung des Hautexpanders angeordnete Bolzen ausgebildet, welche die Haken tragen. Die Fixierelemente können in einfacher Weise als Scheiben ausgebildet sein, die auf die freien Enden der gegenseitig zu fixierenden Träger aufsetzbar sind. Die Bolzen können in eine quer zur Zugrichtung verlaufende Aufnahme der jeweiligen Backen eingelegt werden. Diese Aufnahmen sind in Zugrichtung, d. h. in der der Wunde zugewandten Richtung, offen. Beim Einsatz des Hautexpanders werden die Träger durch die elastische Spannung der Haut in der jeweiligen Aufnahme gehalten. Wenn die einander gegenüberliegenden Träger durch die Fixierelemente in ihrer Position gehalten werden, können die Backen auseinander bewegt werden, sodass die Träger von den Backen freikommen und der Hautexpander abgenommen werden kann.

Vorzugsweise sind die Haken an Hakenmodulen ausgebildet, wobei jedes Hakenmodul nur einen oder einige wenige Haken aufweist. Die Hakenmodule können auf die die Träger bildenden Bolzen aufgeschoben werden, so dass die Anzahl der Haken und ihr gegenseitiger Abstand an dem Träger, d. h. parallel zum Wundrand variabel gewählt werden können.

Im Folgenden wird die Erfindung anhand der in der Zeichnung dargestellten Ausführungsbeispielen näher erläutert. Es zeigen:
- Figur 1: ein Instrument mit aufgesetztem Fixierelement,
- Figur 2: eine Teilansicht dieses Instruments ohne Fixierelement,

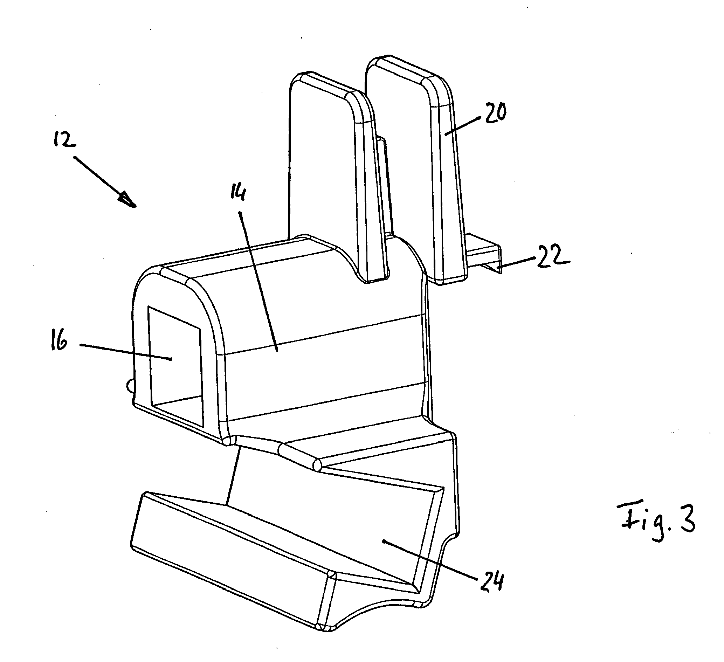

- Figur 3: eine Backe des Instruments,

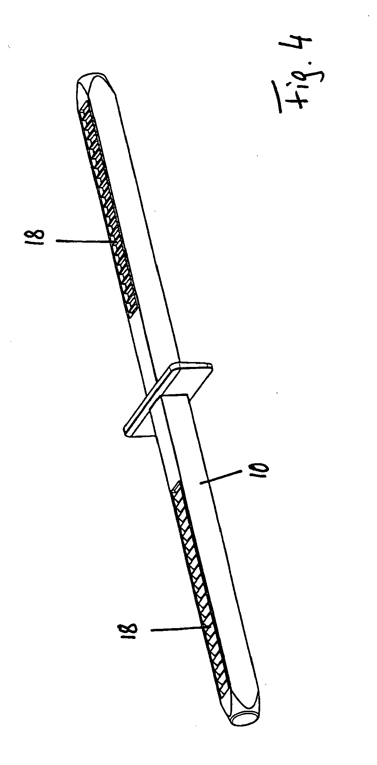

- Figur 4: die Führungsstange des Instruments,
- Figur 5: ein Fixierelement,
- Figur 6: auf einem Träger angeordnete Hakenmodule,
- Figur 7: zwei mittels des Fixierelements fixierte Träger,
- Figur 8: das Fixierelement in einer zweiten Ausführung,
- Figur 9: auf einem Träger angeordnete Hakenmodule in der zweiten Ausführung und
- Figur 10: zwei mittels des Fixierelements fixierte Träger in der zweiten Ausführung.

Das erfindungsgemäße Instrument zum Dehnen der Haut weist einen Hautexpander auf, welcher in der Zeichnung in einer Ausführung dargestellt ist. Andere Ausführungen des Hautexpanders, wie sie aus dem Stand der Technik bekannt sind, können im Rahmen der Erfindung ebenfalls verwendet werden.

Der Hautexpander weist eine langgestreckte gerade Führungsstange 10 auf. Die Führungsstange 10 weist einen unrunden, z. B. rechteckigen Querschnitt auf. Auf der Führungsstange 10 sind längs verschiebbar Backen 12 angeordnet. Die Backen 12 sind auf der Führungsstange 10 längsverschiebbar, wozu sie einen hülsenförmigen Schieber 14 aufweisen, der mit einem Durchbruch 16 auf der Führungsstange 10 geführt ist. Der Innenquerschnitt des Durchbruchs 16 entspricht dem Außenquerschnitt der Führungsstange 10, so dass die Backen 12 unverdrehbar auf der Führungsstange 10 geführt sind. In Längsrichtung der Führungsstange 10 ist eine Rastverzahnung 18 ausgebildet. An dem Schieber 14 ist ein Rasthebel 20 schwenkbar angebracht, der mit einem Rastzahn 22 in die Rastverzahnung 18 eingreift. Die Rastverzahnung 18 ist so ausgebildet, dass die Schieber 14 jeweils von den beiden Enden der Führungsstange 10 gegen die Mitte der Führungsstange 10 hin verschoben werden können, wobei der Rastzahn 22 unter elastischer Auslenkung über die Rastverzahnung 18 gleitet. Der in die Rastverzahnung 18 eingreifende Rastzahn 22 verhindert jedoch, dass die Schieber 14 gegen die Enden der Führungsstange 10 zurückbewegt werden können. Ein Zurückbewegen der Schieber 14 nach außen gegen die Enden der Führungsstange 10 ist nur dann möglich, wenn der jeweilige Rastzahn 22 mittels des Rasthebels 20 aus der Rastverzahnung 18 herausgehoben wird.

Unterhalb des Schiebers 14 weisen die Backen 12 jeweils eine Aufnahme 24 auf. Die Aufnahme 24 hat die Form einer quer zur Führungsstange 10 durchgehend verlaufenden Rinne, die gegen die Vorderseite der Backe 12 hin offen ist. Die Aufnahme 24 hat beispielsweise einen rechteckigen Querschnitt, wobei der rechteckige Querschnitt leicht gegen die zur Führungsstange 10 senkrechte Ebene gekippt ist. Der Kippwinkel kann beispielsweise etwa 30° betragen. Durch diese Kippung liegt die offene vordere Seite der Aufnahme 24 höher, d. h. näher an der Führungsstange 10 als der geschlossene Boden der Aufnahme 24. Die Breite der Aufnahme 24 quer zur Führungsstange 10 entspricht etwa der Breite des Schiebers 14 oder ist etwas breiter als dieser Schieber 14.

In die Aufnahme 24 ist ein Träger 26 einlegbar, an welchem in die Haut einsteckbare Haken 28 angeordnet sind. Der Träger 26 liegt in der Aufnahme 24 mit einem Querschnitt, der dem inneren Querschnitt der Aufnahme 24 entspricht, so dass der Träger 26 mit den Haken 28 unverdrehbar in den Backen 12 gehalten wird. Die Haken 28 sind an den Träger 26 so angeordnet, dass die Haken 28 bei eingelegtem Träger 26 gegen die zur Führungsstange 10 senkrechte Ebene gekippt sind, z. B. um einen Kippwinkel von etwa 30°.

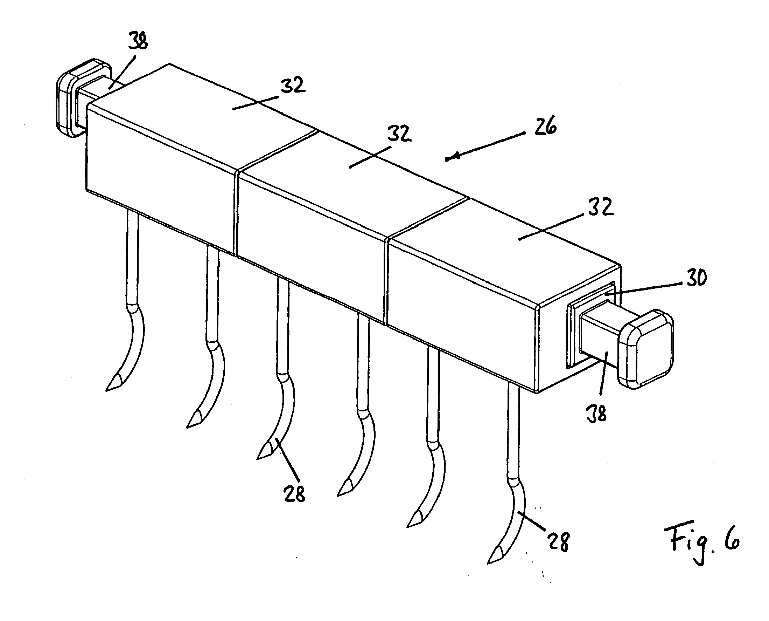

Wie z. B. aus Figur 6 ersichtlich, weist in dem dargestellten Ausführungsbeispiel der Träger 26 einen Bolzen 30 mit unrundem Querschnitt auf. Die Haken 28 sind jeweils in Hakenmodulen 32 angeordnet, die auf den Bolzen 30 axial aufschiebbar sind und aufgrund des unrunden Querschnitts des Bolzens 30 unverdrehbar auf dem Bolzen 30 gehalten werden. Jedes Hakenmodul 32 trägt einen oder einige wenige Haken 28, im dargestellten Ausführungsbeispiel jeweils zwei Haken 28. Die Länge der Bolzen 30 ist vorzugsweise größer als die in Querrichtung verlaufende Länge der Aufnahmen 24, so dass die Träger 26 eine größere Breite als die Backen 12 überdecken. Die Anzahl der auf dem Bolzen 30 aufgesteckten Hakenmodule 32 und deren gegenseitiger Abstand auf dem Bolzen 30 kann frei gewählt werden.

An zumindest einem freien Ende, vorzugsweise an beiden freien Enden des Trägers 26, im dargestellten Ausführungsbeispiel der Bolzen 30 kann jeweils ein Fixierelement 34 aufgesetzt werden. Das Fixierelement 34 hat die Form einer flachen Scheibe, deren Ebene senkrecht zur Querachse der Träger 26, insbesondere senkrecht zur Achse der Bolzen 30 liegt. Die Höhe der Scheibe ist vorzugsweise so gewählt, dass bei aufgesetztem Fixierelement 34 die Haken 28 nach unten über den Umfang des Fixierelements 34 überstehen, während die obere Kante des Fixierelements möglichst wenig über die Backen übersteht. Die Länge der Fixierelemente 34 in Längsrichtung der Führungsstange 10 ist so gewählt, dass das Fixierelement 34 zumindest auf die zwei Träger 26 aufsetzbar ist, die in auf der Führungsstange 10 aneinander angrenzenden Backen 12 aufgenommen sind.

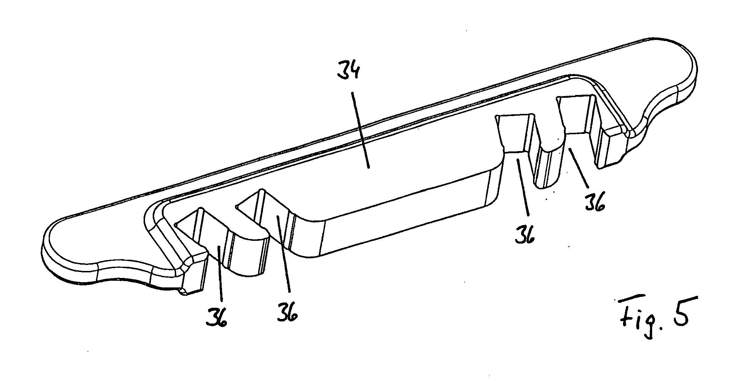

In einer insbesondere in den Figuren 5 bis 7 dargestellten Ausführung weist die Scheibe des Fixierelements 34 an ihrem unteren Rand Randaussparungen 36 auf, mit welchen das Fixierelement 34 von oben auf die freien Enden der Bolzen 30 aufgesteckt werden kann. Die Randaussparungen 36 umgreifen dabei die freien Enden der Bolzen 30 in einem Abschnitt 38 reduzierten Querschnitts. Dadurch werden die Fixierelemente 34 auf den Bolzen 30 gegen eine Verschiebung in Achsrichtung der Bolzen 30 gesichert gehalten. Der Querschnitt der Abschnitte 38 weist unter dem Kippwinkel des Trägers 26 abgeschrägte Parallelflächen auf, denen entsprechend abgeschrägte Parallelflächen der Randaussparungen 36 zugeordnet sind. Das aufgesetzte Fixierelement 34 hält somit die Bolzen 30 und damit die Träger 26 gegen eine Verdrehung um die Achse des Bolzens 30 gesichert.

Nachfolgend wird die Verwendung des erfindungsgemäßen Instruments beschrieben.

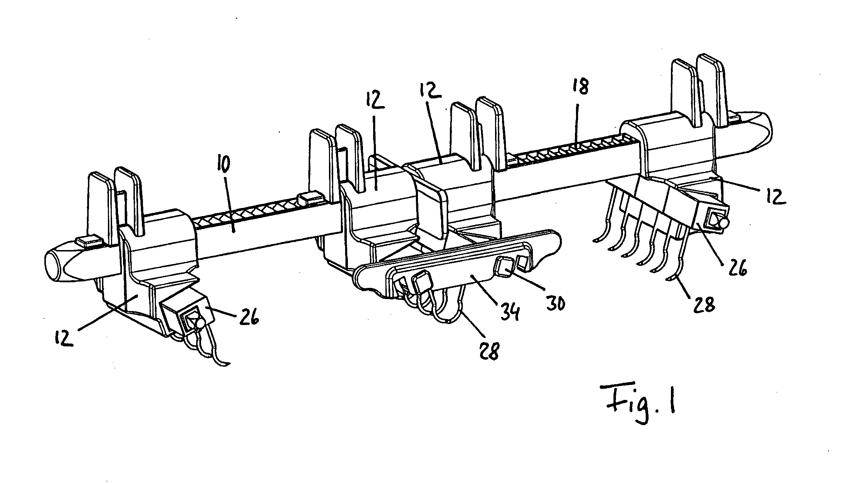

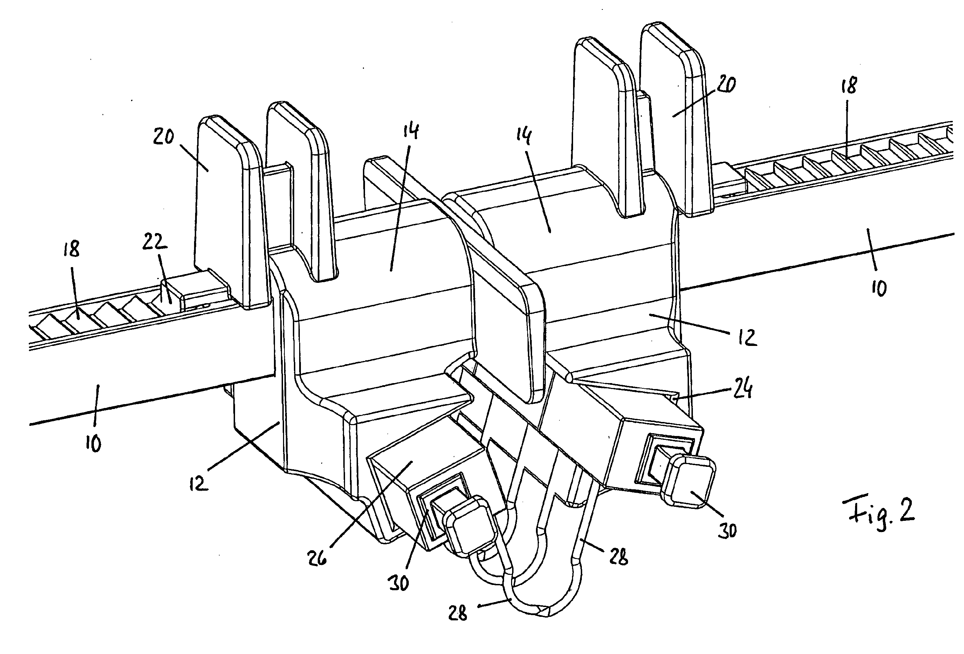

Zum Verschließen eines großflächigen Hautdefektes wird der Hautexpander eingesetzt, wie er beispielsweise in Figuren 1 und 2 dargestellt ist. An den einander gegenüberliegenden Wundrändern wird jeweils ein Träger 26 in der Haut verankert. Hierzu werden insbesondere Bolzen 30 einer geeigneten Länge und einer entsprechend gewählten Anzahl von Hakenmodulen 32 durch Einstechen der Haken 28 in die Haut verankert. Anschließend wird der Hautexpander angesetzt, wobei auf der Führungsstange 10 zwei Backen 12 so angeordnet sind, dass ihre Aufnahmen 24 gegeneinander gerichtet sind. Zunächst sind die Backen 12 mit großem gegenseitigem Abstand auf der Führungsstange 10 positioniert, so dass die an den weit auseinanderliegenden Wundrändern verankerten Träger 26 jeweils in die Aufnahmen 24 der beiden Backen 12 eingelegt werden können. Anschließend werden die Backen 12 auf der Führungsstange 10 gegeneinander bewegt. Dadurch werden die Wundränder gegeneinander gezogen, wobei die in Zugrichtung hinter den Haken 28 liegenden Hautbereiche gedehnt werden. Da die Träger 26 mit den Haken 28 unverdrehbar in der jeweiligen Aufnahme 24 gehalten sind, kann die elastische Kraft der Haut die Haken 28 nicht nach hinten wegkippen und die Träger 26 können nicht aus den Aufnahmen 24 herausgehebelt werden. Da weiter die Haken 28 unter einem Kippwinkel von etwa 30° geneigt sind, wobei die Spitze der Haken 28 nach vorn gerichtet ist, ist auch sichergestellt, dass die Haken 28 nicht aus der Haut des Wundrandes herausgezogen werden. Während die beiden Backen 12 gegeneinander bewegt werden, gleiten die Rastzähne 22 jeweils über die Rastverzahnung 18, während das Einrasten der Rastzähne 22 in die Rastverzahnung 18 verhindert, dass die Backen 12 durch die elastische Zugspannung der Haut zurückgezogen werden.

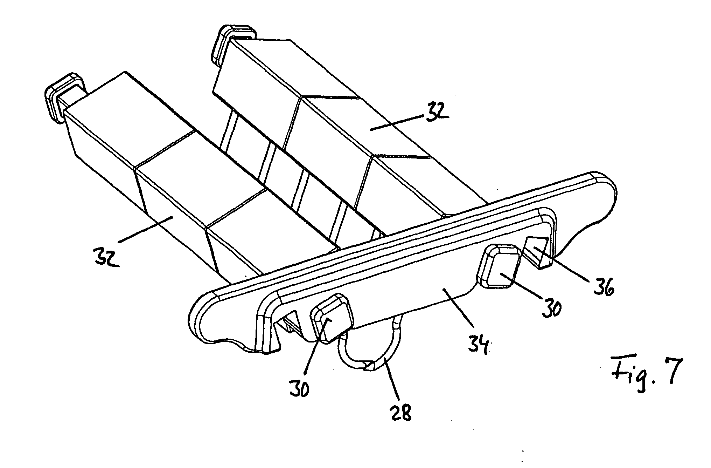

Sobald die beiden Backen 12 vollständig gegeneinander bewegt sind, überlappen sich die gegeneinander gerichteten Spitzen der Haken der 28 geringfügig, z. B. um etwa 3mm, wie dies in Figur 2 gezeigt ist. In dieser Position der Backen 12 wird nun das Fixierelement 34 auf die überstehenden freien Enden der Bolzen 30 aufgesetzt, wie dies in Figur 1 gezeigt ist. Das Fixierelement 34 hält nun die beiden Träger 26 in ihrem Abstand positioniert fest. Vorzugsweise werden zwei Fixierelemente 34 verwendet, wobei jeweils auf beide Enden der Bolzen 30 ein Fixierelement 34 gesetzt wird. Dadurch wird sichergestellt, dass die Bolzen 30 über ihre gesamte Länge positioniert gehalten werden. Die elastische Zugkraft der gedehnten und gespannten Haut zieht die schräg gestellten Bolzen 30 in die schräg gestellten Randaussparungen 36, so dass die Bolzen 30 nicht aus dem Fixierelement 34 frei kommen können. Sobald die Träger 26 durch die Fixierelemente 34 in ihrer Position und in ihrem Abstand fixiert sind, werden die Backen 12 auseinanderbewegt, wozu die Rastzähne 22 mittels der Rasthebel 20 aus der Rastverzahnung 18 herausgehoben werden. Bei dem Auseinanderbewegen der Backen 12 kommen die fixierten Träger 26 aus den Aufnahmen 24 frei, so dass der Hautexpander abgenommen werden kann. Die durch die Fixierelemente 34 zusammengehaltenen Träger 26 mit den Haken 28 (Figur 7) halten die Wundränder aneinander anliegend, so dass ein Vernähen der Wundränder nicht erforderlich ist.

In dem dargestellten Ausführungsbeispiel weist das Fixierelement 34 jeweils zwei Randaussparungen 36 für jeden der Träger 26 auf, wie insbesondere Figur 5 zeigt. Dadurch ist es möglich, die Träger 26 mit den Haken 28 in unterschiedlichem gegenseitigen Abstand zu fixieren, so dass eine optimale Anpassung der aneinander anliegenden Wundränder möglich ist.

Wie in Figur 1 gezeigt ist, können hinter den zwei in den Wundrändern verankerten Backen 12 weitere Backen 12 auf der Führungsstange 10 angeordnet sein, welche Träger 26 aufnehmen, die in einem Abstand von dem jeweiligen Wundrand in dem zu dehnenden Hautbereich mittels Haken 28 verankert werden. Dadurch ist eine gleichmäßigere Verteilung der auf die Haut einwirkenden Zugkraft auf einen größeren Hautbereich möglich, wie dies z. B. aus der EP 2 120 767 B1 bekannt ist.

Falls der Hautdefekt so groß ist, dass er nicht durch eine Hautdehnung in einem Schritt geschlossen werden kann, so können auch Fixierelemente 34 verwendet werden, deren Randaussparung 36 einen größeren Abstand in Zugrichtung aufweisen. Die Backen 12 werden dann jeweils schrittweise gegeneinander bewegt, wobei in jedem Schritt die Haut der Wundränder um einen maximal zulässigen Betrag gedehnt wird. Nach jedem Dehnungsschritt können die Backen 12 in ihrer jeweiligen Position durch geeignet lange Fixierelemente 34 fixiert werden. Der Hautexpander kann dann abgenommen werden, bis er für den nächsten Dehnungsschritt wieder angesetzt wird. Zwischenzeitlich kann die noch offene Wundfläche zusammen mit den in den Wundrändern verankerten und durch die Fixierelemente 34 fixierten Trägern 26 durch eine Vakuumversiegelung abgedeckt werden.

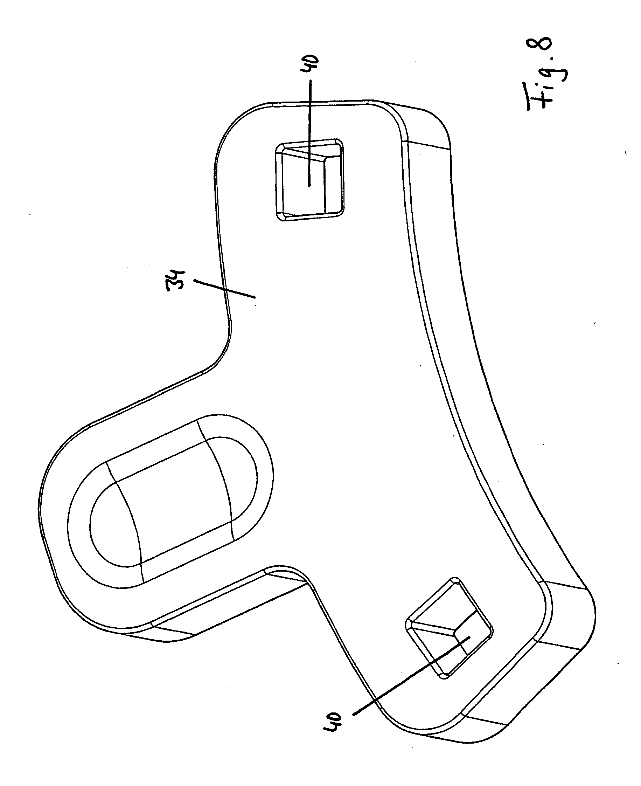

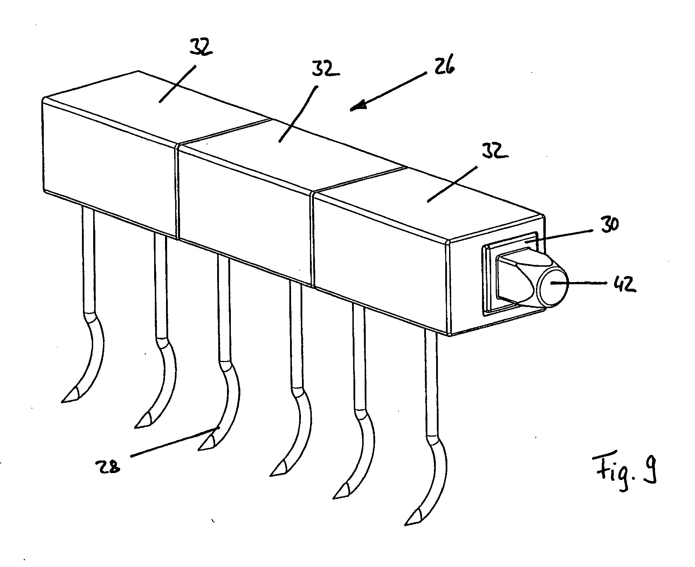

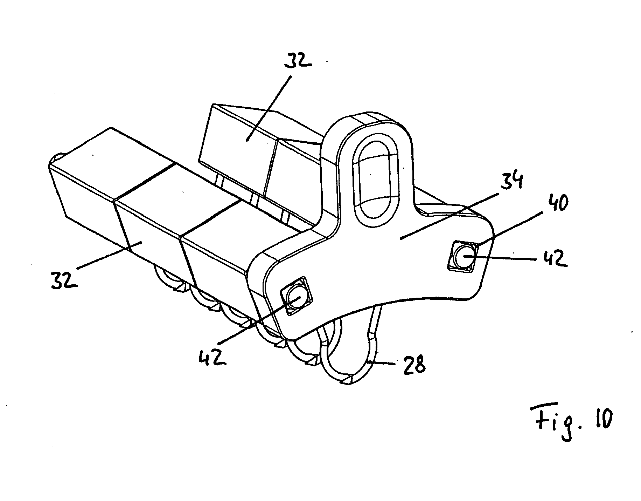

Eine alternative zweite Ausführung des Fixierelements 34 ist in den Figuren 8 bis 10 dargestellt. In dieser Ausführung ist das Fixierelement 34 ebenfalls als flache Scheibe ausgebildet. Die flache Scheibe des Fixierelements 34 weist anstelle der Randaussparungen Durchbrüche 40 in der Fläche des Fixierelements 34 auf, mit welchen das Fixierelement 34 axial auf die freien Enden des Trägers 26, d. h. insbesondere auf die freien Enden 42 der Bolzen 30 axial aufgesteckt werden kann. In dieser Ausführung ist es auch möglich, die Durchbrüche 40 der Fixierelemente 34 als parallel zu der Führungsstange 10 verlaufende Langlöcher auszubilden. Die die Durchbrüche 40 durchgreifenden freien Enden 42 der Träger 26 können in diesen Langlöchern verschoben werden, um den Abstand zwischen den in den beiden Wundrändern verankerten Trägern 26 nachzustellen. Um die Träger 26 in der jeweiligen Nachstellposition zu fixieren, ist in den Langlöchern der Fixierelemente 34 jeweils eine Rastverzahnung angeordnet, die es ermöglicht, die Träger 26 gegeneinander zu bewegen, die aber ein Zurückziehen der Träger 26 durch die elastische Zugkraft der Haut verhindert. Diese Ausführung ermöglicht insbesondere ein Nachstellen der in der Haut verankerten Träger 26, wenn infolge der Hautdehnung die elastische Rückstellzugkraft der Haut nachlässt.

### Bezugszeichenliste

- 10: Führungsstange
- 12: Backen
- 14: Schieber
- 16: Durchbruch
- 18: Rastverzahnung

- 20: Rasthebel
- 22: Rastzahn
- 24: Aufnahme
- 26: Träger
- 28: Haken

- 30: Bolzen
- 32: Hakenmodule
- 34: Fixierelement
- 36: Randaussparungen
- 38: Abschnitt

- 40: Durchbrüche
- 42: freie Enden

## Patentansprüche

1. Instrument zum Dehnen der Haut für den Verschluss von Wunden, mit einem Hautexpander, mit wenigstens zwei an dem Hautexpander mechanisch in einer Zugrichtung gegeneinander verstellbaren Backen (12), mit den Backen (12) zugeordneten Haken (28), die im Bereich der Wundränder in der Haut verankerbar sind, wobei durch Verstellen der Backen (12) über die Haken (28) eine Zugkraft auf die Haut ausgeübt wird, die die Haut dehnt und die Wundränder gegeneinander zieht, wobei
jeweils ein oder mehrere Haken (28) an einem Träger (26) angeordnet sind, der sich quer zur Zugrichtung erstreckt, und wobei die Träger (26) lösbar an den Backen (12) gelagert sind,
**dadurch gekennzeichnet, dass** die Träger (26) durch aufsetzbare Fixierelemente (34) in ihrer gegenseitigen Position fixierbar sind.

2. Instrument nach Anspruch 1,
**dadurch gekennzeichnet, dass** der Träger (26) einen Bolzen (30) aufweist, auf welchem die Haken (28) angeordnet sind.

3. Instrument nach Anspruch 2,
**dadurch gekennzeichnet, dass** jeweils wenigstens ein Haken (28) an einem Hakenmodul (32) angeordnet ist und dass die Hakenmodule (32) auf den einen unrunden Querschnitt aufweisenden Bolzen (30) unverdrehbar aufgesteckt werden.

4. Instrument nach Anspruch 2 oder 3,
**dadurch gekennzeichnet, dass** die Fixierelemente (34) auf die freien Enden der Bolzen (30) aufsetzbar sind.

5. Instrument nach Anspruch 1,
**dadurch gekennzeichnet, dass** die Träger (26) in eine quer zur Zugrichtung verlaufende und in Zugrichtung offene Aufnahme (24) der Backen unverdrehbar einlegbar sind.

6. Instrument nach Anspruch 4,
**dadurch gekennzeichnet, dass** die Fixierelemente (34) als Scheiben ausgebildet sind, die Randaussparungen (36) aufweisen und mit diesen Randaussparungen (36) senkrecht zur Achse der Bolzen (30) auf diese Bolzen (30) aufsteckbar sind.

7. Instrument nach Anspruch 4,
**dadurch gekennzeichnet, dass** die Fixierelemente (34) als Scheiben ausgebildet sind, die Durchbrüche (40) aufweisen und mit diesen Durchbrüchen (40) axial zu den Bolzen (30) auf diese Bolzen aufsteckbar sind.

8. Instrument nach einem der vorhergehenden Ansprüche,
**dadurch gekennzeichnet, dass** die Träger (26) in veränderbarem gegenseitigen Abstand durch die Fixierelemente (34) fixierbar sind.

9. Instrument nach Anspruch 6 und 8,
**dadurch gekennzeichnet, dass** die Fixierelemente (34) in Zugrichtung gegeneinander versetzte Randaussparungen (36) aufweisen, mit welchen sie wahlweise auf die Bolzen (30) aufsteckbar sind.

10. Instrument nach Anspruch 7 und 8,
**dadurch gekennzeichnet, dass** die Durchbrüche (40) eine in Zugrichtung verlaufende Rastung aufweisen und dass die Bolzen (30) in dieser Rastung in Zugrichtung verstellbar einrastbar sind.

## Claims

1. An instrument for stretching the skin for closing wounds, with a skin expander, with at least two jaws (12) mechanically adjustable with respect to each other in a tensioning direction on the skin expander, with hooks (28) which are associated with the jaws (12) and which are capable of being anchored in the skin in the region of the edges of the wound, wherein a tensile force is exerted on the skin by way of the hooks (28) by adjustment of the jaws (12), the said tensile force stretching the skin and drawing the edges of the wound towards one another, wherein one or more hooks (28) is or are arranged in each case on a carrier (26) which extends transversely to the tensioning direction, and wherein the carriers (26) are mounted on the jaws (12) in a releasable manner, **characterized in that** the carriers (26) are capable of being fixed in position with respect to each other by fixing elements (34) capable of being attached.

2. An instrument according to claim 1, **characterized in that** the carrier (26) has a pin (30) on which the hooks (28) are arranged.

3. An instrument according to claim 2, **characterized in that** at least one hook (28) is arranged in each case on a hook module (32) and the hook modules (32) are set in a non-rotatable manner on the pin (30) which has a non-round cross-section.

4. An instrument according to claim 2 or 3, **characterized in that** the fixing elements (34) are capable of being set on the free ends of the pins (30).

5. An instrument according to claim 1, **characterized in that** the carriers (26) are capable of being inserted in a non-twist manner into a receiving means (24) in the jaws extending transversely to the tensioning direction and open in the tensioning direction.

6. An instrument according to claim 4, **characterized in that** the fixing elements (34) are designed in the form of plates which have edge recesses (36) and are capable of being set on the pins (30) with these edge recesses (36) at a right angle to the axis of these pins (30).

7. An instrument according to claim 4, **characterized in that** the fixing elements (34) are designed in the form of plates which have apertures (40) and are capable of being set on the pins (30) with these apertures (40) axially with respect to these pins.

8. An instrument according to any one of the preceding claims, **characterized in that** the carriers (26) are capable of being fixed at a variable mutual distance by the fixing elements (34).

9. An instrument according to claim[s] 6 and 8, **characterized in that** the fixing elements (34) have edge recesses (36) which are offset from one another in the tensioning direction and by which they are optionally capable of being set on the pins (30).

10. An instrument according to claim[s] 7 and 8, **characterized in that** the apertures (40) have a catch extending in the tensioning direction and the pins (30) are capable of engaging in this catch in the tensioning direction.

## Revendications

1. Instrument pour dilater la peau pour fermer les cicatrices comportant un expanseur de peau ayant au moins deux mors (12) réglables l'un par rapport à l'autre mécaniquement dans une direction de traction, les mors (12) comportant des crochets (28) qui s'accrochent dans la région des bords de la cicatrice,
le réglage des mors (12) par les crochets (28) permettant d'exercer une force de traction sur la peau qui étire la peau et tirant l'un vers l'autre les bords de la cicatrice, chaque fois un ou plusieurs crochets (28) étant prévus sur un support (26) qui s'étend transversalement à la direction de traction, et
les supports (26) étant montés mobiles sur les mors (12), instrument **caractérisé en ce que**
les supports (26) peuvent être bloqués dans leur position réciproque par des éléments de fixation rapportés (34).

2. Instrument selon la revendication 1,
**caractérisé en ce que**
le support (26) comporte un goujon (30) muni des crochets (28).

3. Instrument selon la revendication 2,
**caractérisé en ce que**
chaque fois au moins un crochet (28) est installé sur un module de crochets (32) et les modules de crochets (32) sont engagés de manière solidaire en rotation sur un goujon (30) à section non circulaire.

4. Instrument selon la revendication 2 ou 3,
**caractérisé en ce que**
les éléments de fixation (34) sont rapportés aux extrémités libres des goujons (30).

5. Instrument selon la revendication 1,
**caractérisé en ce que**
les supports (26) sont logés de manière rotative dans un logement (24) ouvert dans la direction de traction et dirigés transversalement à la direction de traction des mors.

6. Instrument selon la revendication 4,
**caractérisé en ce que**
les éléments de fixation (34) sont en forme de plaques, munis d'encoches (36) au bord et qui s'engagent sur les goujons (30) par ces encoches du bord (36) perpendiculairement à l'axe des goujons (30).

7. Instrument selon la revendication 4,
**caractérisé en ce que**
les éléments de fixation (34) en forme de plaques, munis de passage (40) et s'engagent sur les goujons par les passages (40) axialement aux goujons (30).

8. Instrument selon l'une des revendications précédentes, **caractérisé en ce que**
les supports (26) sont fixés à intervalles réciproques variables par les éléments de fixation (34).

9. Instrument selon les revendications 5 et 6,
**caractérisé en ce que**
les éléments de fixation (34) ont des encoches de bord (36) décalées les unes par rapport aux autres dans la direction de traction et par lesquelles ils s'engagent sélectivement sur les goujons (30).

10. Instrument selon les revendications 7 et 8,
**caractérisé en ce que**
les passages (40) ont un moyen d'encliquetage dans la direction de traction et les goujons (30) sont encliquetables de manière réglables dans ce moyen d'encliquetage selon la direction de traction.
